Europäisches Patentamt

European Patent Office

Office européen des brevets

(19)

(11) Veröffentlichungsnummer: **0 175 195 B1**

(12) **EUROPÄISCHE PATENTSCHRIFT**

(45) Veröffentlichungstag der Patentschrift: **04.12.91**

(51) Int. Cl.⁵: **G01N 33/52**, //G01N33/545, G01N33/546,C12Q1/00

(21) Anmeldenummer: **85111004.9**

(22) Anmeldetag: **31.08.85**

(54) **Vorrichtung aus Polymeren mit Membranstruktur und eingelagerten Feststoffpartikeln.**

(30) Priorität: **13.09.84 DE 3433563**

(43) Veröffentlichungstag der Anmeldung:
**26.03.86 Patentblatt 86/13**

(45) Bekanntmachung des Hinweises auf die Patenterteilung:
**04.12.91 Patentblatt 91/49**

(84) Benannte Vertragsstaaten:
**AT BE CH DE FR GB IT LI LU NL SE**

(56) Entgegenhaltungen:
**EP-A- 0 016 387**
**WO-A-79/01081**
**DE-A- 2 739 008**
**US-A- 3 993 451**
**US-A- 4 356 149**

(73) Patentinhaber: **BEHRINGWERKE Aktiengesellschaft**
**Postfach 1140**
**W-3550 Marburg 1(DE)**

(72) Erfinder: **Scharf, Gerhard**
**Wiesenhofstr. 15**
**W-3560 Biedenkopf-Kombach(DE)**

(74) Vertreter: **Becker, Heinrich Karl Engelbert, Dr. et al**
**HOECHST AKTIENGESELLSCHAFT Central Patent Department P.O. Box 80 03 20**
**W-6230 Frankfurt am Main 80(DE)**

## Beschreibung

Die Erfindung betrifft eine Vorrichtung von vorzugsweise flächenförmiger Gestalt bestehend aus einem wasserfesten Polymeren mit Membranstruktur und eingelagerten Feststoffpartikeln, die mindestens einen Teilnehmer einer chemischen Reaktion enthält. Eine solche Vorrichtung kann als analytisches Mittel und besonders als Testvorrichtung vorzugsweise als Teststreifen verwendet werden.

Die Verwendung von membranfilter-ähnlichem Material für die Herstellung von Testmitteln, wobei die Membrane eine reine Papier-Ersatzfunktion hat, und wobei Füllstoffe, auch solche mit adsorbierenden Eigenschaften eingelagert werden können, ist in der deutschen Offenlegungsschrift 26 02 975 beschrieben.

Aus der deutschen Offenlegungsschrift 19 56 214 ist bekannt, Cellulose, vernetzte Dextrane oder Ionenaustauscher als Reagenzienträger in organischen Lösungen von Polymeren wie Ethylcellulose oder Celluloseacetat zu suspendieren und damit Kunststoffträger zu beschichten. Die Nachteile einer derartigen Methode bestehen darin, daß die Saugfähigkeit der Reagenzienträger stark herabgesetzt wird. Diese sind in einem Kunststoffilm eingelagert, wobei die Porenweite des Films durch den Herstellungsprozess nur ungenügend steuerbar ist. Außerdem können die Poren zum Beispiel bei Cellulose als eingelagertem Material beim Trocknen der Filme verschlossen werden.

In der deutschen Offenlegungsschrift 30 07 744 werden Testreagenzien in der Form von modifizierten Cellulose-Materialien in organischen Polymer-Lösungen, welche die Funktion eines Klebers haben, suspendiert und auf Plastikträger aufgebracht.

Große Schwierigkeiten bereiten diese Techniken, wenn beispielsweise mehrere Zonen von unterschiedlichen Feststoff-Materialien hergestellt werden sollen, die im Sinne eines chromatographischen Prozesses successive mit Laufmittel durchströmt werden sollen. Hierbei müssen die Zonen eine hohe Saugfähigkeit haben und sich berühren, und die Reaktionsfähigkeit darin enthaltener Reagenzienträger muß erhalten bleiben.

In der deutschen Offenlegungsschrift 29 22 856, welche einen Nachweis von Glukose betrifft, werden beispielsweise auf einem Papierstreifen durch Auftragen mittels Federn verschiedene Reagenzienlösungen parallel aufgetragen.

In der deutschen Offenlegungsschrift 26 20 923 wird das Problem, mehrere Zonen saugfähiger Materialen einander berührend anzuordnen derart gelöst, daß die Materialien successive in kleine Glassäulen eingefüllt werden.

Die Unzulänglichkeit der bisherigen Aufbring-Methoden wird besonders an diesem letzten Verfahren deutlich. Da dort durch Farbveränderung einer chromogenen Zone beim Durchströmen eines Analyten eine substanzproportionale Farbzone erhalten werden soll, müssen die Zonen eine hohe Saugfähigkeit besitzen, und ihre Dichte muß bei der Herstellung reproduzierbar sein.

Sollen für Testmittel mit den beschriebenen Aufgabenformen Teststäbchen verwendet werden, müssen die aufgebrachten Schichten eine hohe Saugfähigkeit haben und die Poren des gegebenenfalls porösen Feststoffs dürfen durch den Herstellungprozess nicht verschlossen werden. Diese Voraussetzungen sind mittels der angegebenen Methoden nicht gewährleistet; insbesondere betrifft dies die Saugfähigkeit.

Um die Saugfähigkeit von Polymer-Filmen zu regeln, wird in der deutschen Oftenlegungsschrift 29 10 134 derart verfahren, daß in Polymer-Filme bei ihrer Herstellung Feststoffpartikel eingelagert werden. Die Filme werden dadurch "geöffnet", daß der Gehalt der "Filmöffner" die Saugfähigkeit dosiert. Werden aber saugende Feststoffpartikel wie Cellulose eingelagert, beobachtet man, daß die Saugfähigkeit, etwa verglichen mit derselben Menge von eingelagertem Gips stark abnimmt. Es ist hier davon auszugehen, daß zumindest teilweise die Poren der Cellulose dabei verschlossen werden, und daß das filmbildende Polymere mit der Oberfläche der Cellulose verklebt.

Verwendet man als "Filmöffner" eine Cellulose, welche gemäß der deutschen Offenlegungschrift 30 16 618 zu einem polymeren Enzymsubstrat für Peroxydase modifiziert wurde, so ist beim Eintauchen in eine Lösung, welche Wasserstoffperoxyd sowie Peroxydase enthält nur eine sehr langsame Reaktion erkennbar. Um die Reaktionsfähigkeit der normalen Cellulose-Präparate annähernd zu erreichen, muß der Polymer-Gehalt der filmbildenden Suspension so weit abgesenkt werden, daß eine hinreichende mechanische Stabilität der gebildeten Schicht nicht mehr gewährleistet ist.

Die vorliegende Erfindung hatte sich daher die Aufgabe gestellt, eine Zubereitungsform von Feststoffen mit gekoppelten Reaktionssystemen zu entwickeln, welche die beschriebenen Nachteile überwindet.

In diesen Zubereitungsformen soll ein Bestandteil die für praktische Zwecke erforderliche Festigkeit gewährleisten, während ein weiterer eingelagerter Bestandteil mindestens eine Substanz enthält oder trägt, die ein Teilnemer an einer chemischen Reaktion sein kann. Als Stoffe, welche dem System Festigkeit geben sollen, sind im Rahmen der Erfindung membranbildende Stoffe gemeint, besonders wasserfeste Kunststoffe mit Membranfilterstruktur.

Vorzugsweise hat eine solche Zubereitungsform eine flächenförmige Gestalt.

Gegenstand der Erfindung ist daher eine vorzugsweise flächenförmige Vorrichtung bestehend aus in ein wasserfestes Polymer mit Membranfilter-Struktur eingelagerten Feststoffpartikeln, und enthaltend einen oder mehrere Teilnehmer einer chemischen Reaktion, dadurch gekennzeichnet, daß mindestens ein Reaktionsteilnehmer an Feststoffpartikel gebunden ist.

Bevorzugt bestehen die eingelagerten Feststoffpartikel aus einem saugfähigen Material wie Cellulose oder Cellulosederivate, vernetzte Dextrane, Ionenaustauscher, Polyamide, poröse Gläser, makroporöse Polymere, Zeolithe, Kieselgur, poröses Siliciumdioxid, Kaolin oder Aktivkohle.

Das beschriebene Verfahren zur Herstellung einer solchen Vorrichtung erhält die Saugfähigkeit der eingelagerten Partikel.

Dabei kann es sich bei diesen Feststoffpartikeln auch um Partikel mit nicht saugendem Kern handeln.

Methoden zur Bindung von Partnern einer chemischen Reaktion an Feststoffe sind bekannt. Diese Methode betreffen rein adsorptiv gebundene Systeme wie beispielsweise bereits 1916 für die Adsorption von Enzymen an Kohle und Aluminium beschrieben (Weetall, H.H., Anal. Chem. 46, 602 A (1974).

Weitere brauchbare Materialien sind Aluminiumoxyd, Tonerde oder Glas.

Weiterhin sind Methoden bekannt, um an derartige Feststoffe chemische Reaktanten wie z.B. Säure-Base-Indikatoren, Chelatbildner, biologisch wirksame Substanzen wie Enzyme, Antigene oder Antikörper kovalent zu binden. Man kann beispeisweise durch Reaktion mit Aminopropyl-triethoxysilan Aminopropyl-Derivate von Gläsern erhalten, welche sich mit einer Vielzahl von Methoden mit Stoffen umsetzen lassen, die dann weiter als Partner einer chemischen Reaktion fungieren können.

Weiterhin gibt es Feststoffe, bei denen auf einem festen Kern eine Serie von adsorbierten Mikropartikeln fixiert sind. Diese Mikropartikel können analog zu in Membranen eingelagerten Reaktionssystemen verwendet werden.

Derartige reagenztragende nicht- oder teilweise saugende Feststoffpartikel können gemäß der vorliegenden Erfindung zu einem mechanisch stabilen Material verarbeitet werden, wobei die Saugfähigkeit und Reaktionsfähigkeit erhalten bleiben.

Die beschriebenen Produkte oder Vorrichtungen können hergestellt werden, indem Polymere in Lösungsmittelsystemen gelöst werden, welche niedrigsiedende Lösungsmittel für das Polymer, höhersiedende Quellmittel sowie Fällungsmittel für das Polymer enthalten. Dabei ist es wesentlich, daß die Fällungsmittel bevorzugt von dem einzulagernden Feststoff absorbiert werden.

Wird in einem derartigen Polymer-Lösungmittelsystem ein Feststoff suspendiert und die Suspension auf eine Unterlage ausgegossen, bildet sich während der Verdunstung des niedrigsiedenden Polymer-Lösungsmittels ein Koazervat, wobei die Koagulation in der Nähe der mit Fällungsbestandteilen bevorzugt beladenen Feststoffpartikel beginnt. Dadurch wird erreicht, daß das ausfallende Polymere nicht mit den Feststoffpartikeln verkleben kann. Man erhält ein Produkt, bei welchem das Polymere eine hohlschaumartige Struktur besitzt, wobei die Feststoffpartikel von dem Polymeren umhüllt werden.

Gegenstand der Erfindung ist daher weiterhin ein Verfahren zur Herstellung einer vorstehend beschriebenen Vorrichtung, dadurch gekennzeichnet, daß Feststoffpartikel mit einer membranfilterbildenden Polymerlösung vermischt und wasserfeste Membranen hergestellt werden.

Bevorzugt ist ein Verfahren unter Verwendung von saugfähigen Feststoffpartikeln, dadurch gekennzeichnet, daß Feststoffpartikel mit einer membranfilterbildenden Polymerlösung vermischt werden, wobei das Lösungsmittelsystem so gewählt wird, daß die fällenden Anteile des Lösungsmittelgemisches bevorzugt in die saugfähigen Feststoffpartikel eindringen, und Membranen hergestellt werden.

Der Anteil des wasserfesten Polymeren am Gewicht der Gesamtzubereitung kann von 3 bis 50 g/100 g betragen.

Besonders bevorzugt ist ein Verfahren, worin ein Partner einer chemischen Reaktion an die Feststoffpartikel gebunden ist oder gebunden wird.

Dieses Verfahren gestattet die Herstellung von sich berührenden Zonen mit gegebenenfalls unterschiedlichen Feststoffmaterialien, wobei dann Produkte erhalten werden, welche sich für einen chromatographie-ähnlichen Durchsatz von Flüssigkeiten eignen, wobei die sich berührenden Zonen nacheinander durchströmt werden können.

Eine derartiges Produkt ist beispielsweise für die Herstellung eines störungsfreien Harnzuckertests brauchbar. Hierzu können die verschiedenen Zonen die folgenden Funktionen haben: Zone 1 enthält Ionenaustauscher, welche mit den Bestandteilen eines Puffersystems beladen sind; Zone 2 enthält ein polymeres Enzymsubstrat auf der Basis von an Cellulose-gekoppeltem o-Dianisidin sowie die für die Reaktionen erforderlichen Enzyme.

Wird ein auf diese Weise hergestellter Teststreifen in Glukose-haltigen Harn getaucht, werden störende Bestandteile wie Ascorbinsäure in der Zone 1 entfernt und gleichzeitig die gebundenen Puffersubstanzen freigesetzt, worauf sich beim Durchströmen der Zone 2 eine Oxidation der Glukose und des an ein Polymeres gebundenen Chromogens durch Peroxydase anschließt.

Mittels der bekannten Techniken sind derartige Produkte mit ausreichender Gebrauchsstabilität nicht herstellbar.

Das erfindungsgemäße Verfahren ergibt Produkte, in denen Feststoffpartikel in hohlschaumartige wasserfeste Polymere eingeschlossen sind, wobei die Saugfähigkeit gegebenenfalls saugfähiger Partikel erhalten bleibt und gegebenenfalls ein Partner einer chemischen Reaktion an diese Partikel gebunden sein kann.

Die Produkte haben bei Polymer-Gehalten zwischen 3 und 50, vorzugsweise 6 und 35 g/100 g des insgesamt aufgebrachten Materials mach Trocknung eine sehr gute mechanische Stabilität.

Des weiteren kann man durch Ausgießen der Feststoff-Polymer-Suspensionen auf Kunststoffträger erreichen, daß das membranfilterbildende Polymere mit der Unterlage verschweißt wird.

Durch Ausgießen von mehreren parallelen, sich berührenden Zonen können Testmittel erhalten werden, welche sich als Problemlösungen der oben beschriebenen chromatographie-ähnlich betriebenen Systeme eignen.

Die beschriebene Technik erlaubt weiterhin die Herstellung mehrschichtiger Elemente. Insbesondere können bei Verwendung transparenter Träger auf der chromogenen Zone, die sich auf dem Träger befindet, weitere Schichten mit filternden, lichtreflektierenden oder ionenaustauschenden Funktionen aufgebracht werden.

Es war überraschend, daß sich Feststoffe, die Reaktanden tragen, derart in Membrane einarbeiten ließen, daß Saugfähigkeit und Reaktionsfunktion erhalten bleiben.

Dies ist beispielsweise für den Fall einer Einlagerung von Cellulose-Derivaten in membranfilterbildende Polymer-Lösungen keineswegs selbstverständlich. Systeme auf der Basis gewisser Cellulose-Derivate als membranfilterbildende Polymere liefern in gebräuchlichen Lösungsmittel-Systemen bestehend aus Aceton/Alkoholen/Wasser in vielen Fällen mit Cellulose als Füllstoff kaum saugende Produkte. Senkt man den Polymergehalt, um die Saugfähigkeit zu erhalten, zeigen die Produkte eine ungenügende mechanische Stabilität.

Um eine hinreichende Absorption der Fällungsbestandteile durch den Feststoff zu gewährleisten, ist neben einer hinreichenden Differenzierung der Affinität der Lösungsmittel-Bestandteile zu dem Feststoff die Wahl eines strukturell ausreichend unterschiedlichen membranfilterbildenden Polymeren wesentlich. Bei Cellulose eignen sich hydrophobe Polymere beispielsweise besonders PVC oder Polystyrol.

Hydrophobe Polymere wie PVC können dabei neben dem allgemeinen Zusatz von Tensiden durch Zusatz von mit dem Polymeren mischbaren oberflächenaktiven Substanzen bezüglich ihres Benetzungsverhaltens wesentlich modifiziert werden. Beispielsweise kann dies bei PVC durch Zusatz von niedermolekularem Polyoxethylen oder Trialkylmethylammoniumhalogeniden erfolgen.

Ganz allgemein sind Saugfähigkeit und Abriebfestigkeit durch voneinander unabhängige Maßnahmen steuerbar. Für die Saugfähigkeit ist sowohl die Porenweite und eine gut ausgebildete Membranfilterstruktur als auch die Saugfähigkeit des Feststoffpartikel wesentlich. Die Saugfähigkeit des Produktes kann weiterhin durch einen Zusatz eines Detergenzes verbessert werden. Das Detergenz kann in einer Menge von 0 bis 25 g/100 g des Produktes verwendet werden.

Die Abriebfestigkeit ist naturgemäß von dem Anteil des eingelagerten Feststoffs an der Gesamtmasse abhängig. Eine Rolle spielen aber auch die Güte der Membranfilterstruktur, die Gummielastizität sowie die Flexibilität des Polymeren wie auch die Aufbringstärke und eine möglicherweise stattfindende Sedimentation des Feststoffs beim Verdunstungsvorgang.

Gut ausgebildete Membranfilterstrukturen können auch ohne eingelagerte Feststoffe für reine Transportfunktionen in Kombination mit erfindungsgemäßen Vorrichtungen verwendet werden.

Insbesondere kann bei der Aufbringung von mehreren sich berührenden Zonen eine Zone zur Definition des Ansaugvolumens eines Analyten verwendet werden. Hier hätten eingelagerte hydrophile Feststoffe möglicherweise den Nachteil, daß deren Ansaugkapazität vom Trocknungsgrad, das heißt von der Luftfeuchtigkeit und der Umgebungstemperatur abhängt.

Membranfilterzonen lassen sich naturgemäß mit beliebigen anderen sich berührenden Zonen z.B. aus Papier, Fibern, Geweben zu Systemen verarbeiten, deren Anwendung innerhalb chromatographieartiger Prozesse von Bedeutung sein kann.

In der Folge wird die Erfindung mittels einiger nicht einschränkende Beispiele erläutert.

Beispiel 1

Es wurden 0,25 g Diethylamino-Cellulose in 0,025 ml Wasser, 0,1 ml Essigsäureethylester und 1 ml Tetrahydrofuran suspendiert. Dann wurden 0,3 g einer 10 gewichts-%igen PVC-Lösung in Tetrahydrofuran zugegeben. Nach Homogenisieren wurde die Suspension auf eine Glasplatte gegossen und zu einer Dicke von 300 Mikrometer ausgestrichen. Nach dem Trocknen erhielt man eine gut saugende Membrane mit integriertem Cellulose-Material.

Beispiel 2

Es wurde analog Beispiel 1 eine Suspension hergestellt und ebenfalls in der Dicke 300 μm auf eine 0,5 mm starke PVC-Folie aufgebracht. Das Material erwies sich nach dem Trocknen als gut saugend und mit dem Träger verschweißt. Die aufgeschweißte Membrane ist im trockenen und im feuchten Zustand abriebfest. Zur Untersuchung der Struktur wurden die auf PVC aufgeschweißten Membranen elektronenmikroskopisch untersucht. Die Aufnahmen, insbesondere die Element-Verteilungsaufnahmen bezüglich des Elementes Chlor mit einem energiedispersiven Röntgenspektrometer bestätigten die vermutete PVC-Membranstruktur.

Beispiel 3

Gemäß der deutschen Auslegeschrift 30 16 618, Beispiel 1, wurde auf der Basis von Cellulose als Träger und Tetramethylbenzidin als Chromogen ein polymeres Chromogen als Enzymsubstrat des System Peroxydase/Wasserstoffperoxyd hergestellt.
0,25 g dieses polymeren Chromogens wurden mit 0,05 g Wasser, 0,1 ml absolutem Alkohol, 0,9 ml Tetrahydrofuran sowie 0,1 ml einer 10 volumen-%igen Lösung von Polyethylenglykol 400 in Tetrahydrofuran suspendiert, und es wurde dann 0,25 g einer 7 gewichts-%igen Lösung von PVC in Tetrahydrofuran zugegeben und die Suspension nach 5 Minuten homogenisiert. Dann wurde die Suspension in einer Dicke von 300 μm auf einen PVC Träger aufgerakelt. Nach dem Austrocknen erhielt man eine gut saugende auf den Träger aufgeschweißte Membrane. Beim Eintauchen in eine Lösung von pH 5, welche 0,1 Volumen-% Wasserstoffperoxyd und etwas Peroxydase enthält, wurde das in die Membrane integrierte polymere Chromogen unter Bildung einer grünen Farbe oxydiert.

Beispiel 4

Gemäß der deutschen Offenlegungsschrift 19 56 214, Beispiel 19, wurde diazotierte p-Aminobenzyl-Cellulose hergestellt. Die salzsaure Lösung wurde bei 0°C unter starkem Rühren mit kleinen Portionen Natriumacetat versetzt, bis der pH-Wert etwa 4,5 betrug. Nun wurde eine volumengleiche 5 volumen-%ige Lösung von N-N-Dimethylanilin in N,N-Dimethylformamid, welche vorher auf 0°C abgekühlt wurde, zugegeben und die Mischung 30 Minuten im Eisbad gerührt. Nun wurde abgesaugt und das Cellulose-Produkt extensiv mit Wasser gewaschen.
Das Material erwies sich als ein polymerer, nicht ausblutender Säure-Base-Indikator, welcher bei Säure-Zusatz von gelb nach rot umschlägt.
Das getrocknete Material wurde gemäß Beispiel 1 mittels einer Stahlschablone der Dicke 250 μm die ein Andreaskreuz zeigte, auf einen gelb pigmentierten Aufkleber aus PVC aufgebracht. Nach dem Austrocknen erzeugte säurehaltiges Wasser ein rotes Warnkreuz.

Beispiel 5

Zur Anwendung des polymeren Säure-Base Indikators aus Beispiel 4 zur Bestimmung von pH-Werten wurde ein weiß pigmentierter PVC-Träger mittels eines Beschichtungsgerätes mit sieben parallelen Streifen der Breite 3 mm, zwischen denen sich jeweils ein Abstand von 4 mm befand, beschichtet. Die Zwischenzonen wurden mit PVC-Farben in einer Breite von 3 mm beschichtet, wobei die PVC-Farben abgestuft in 0,2 pH-Schritten den Umschlagsfarben des Indikators im Bereich 4,6 bis 3,4 angepaßt waren.
Nach dem Austrocknen wurde der Träger senkrecht zu den Farbzonen in Streifen geschnitten.
Dieser nicht ausblutende Indikator-Teststreifen bietet Vorteile gegenüber den bisherigen Produkten bei der pH-Zuordnung. Durch die eng benachbarten Farbzonen ähnlicher Farb- und Oberflächenstruktur ist die subjektiv erreichbare Zuordnungsgenauigkeit gegenüber den bisherigen Teststreifen erhöht.

Beispiel 6

Es wurden 0,15 g eines Cellulose-Präparates, auf welches durch substantive Färbung Kongorot aufgezogen worden war, in einer Mischung von 0,3 g 10 Gewichts-%iges PVC in Tetrahydofuran, 0,6 ml Tetrahydrofuran und 0,2 ml Alkohol suspendiert und in einer Stärke von 400 μm auf einen Träger aus Hart-PVC aufgebracht.
Nach dem Abdunsten der Lösungsmittel erhielt man eine gut saugende und weitgehend abriebfeste Aufbringung, welche sich als weitgehend ausblutsichere Säure-Base-Indikatur verwenden läßt.

**Patentansprüche**

1. Verfahren zum Herstellen einer Vorrichtung, die aus einer wasserfesten polymeren Membranfilterstruktur mit darin eingelagerten Feststoffpartikeln besteht und die einen oder mehrere Teilnehmer einer chemischen Reaktion und gegebenenfalls ein Detergenz enthält, dadurch gekennzeichnet, daß die Feststoffpartikel mit einer membranfilterbildenden Polymerlösung vermischt werden, wobei die Polymere in Lösungsmittelsystemen gelöst werden, welche niedrig siedende Lösungsmittel für das Polymer, höher siedende Quellmittel sowie Fällungsmittel für das Polymer enthalten.

2. Verfahren gemäß Anspruch 1, dadurch gekennzeichnet, daß die fällenden Anteile des Lösungsmittelgemisches so gewählt werden, daß die fällenden Anteile des Lösungsgemisches bevorzugt in die saugfähigen Feststoffpartikel eindringen.

3. Vorrichtung bestehend aus einer Membranfilterstruktur aus einem wasserfesten Polymer mit eingelagerten Feststoffpartikeln enthaltend einen oder mehrere Teilnehmer einer chemischen Reaktion und gegebenenfalls ein Detergenz, dadurch gekennzeichnet, daß sie mit einem Verfahren nach einem der Ansprüche 1 und 2 hergestellt worden ist.

4. Vorrichtung nach Anspruch 3, dadurch gekennzeichnet, daß die eingelagerten Feststoffpartikel saugfähig sind.

5. Vorrichtung nach Anspruch 3, dadurch gekennzeichnet, daß sie flächenförmig ist.

6. Vorrichtung nach Anspruch 3, dadurch gekennzeichnet, daß der Anteil des wasserfesten Polymeren von 3 bis 50, vorzugsweise 6 bis 35 g/100 g Vorrichtung beträgt

7. Vorrichtung nach Anspruch 3, dadurch gekennzeicnhnet, daß der Anteil an Feststoffpartikeln 50 bis 97, vorzugsweise 65 bis 94 g/100 g Vorrichtung beträgt.

8. Vorrichtung nach Anspruch 3, dadurch gekennzeichnet, daß sie 0 bis 25 g eines Detergenz pro 100 g Vorrichtung enthält.

## Claims

1. A process for preparing a device which is composed of a waterproof polymeric membrane filter structure with solids particles incorporated therein and which contains one or more participants in a chemical reaction and, if appropriate, a detergent, which process is characterized in that the solids particles are mixed with a polymer solution which forms membrane filters, the polymers are dissolved in solvent systems which contain low-boiling solvents for the polymer, higher-boiling swelling agents and precipitants for the polymer.

2. The process as claimed in claim 1, wherein the precipitating fractions of the solvent mixture are selected such that the precipitating fractions of the solution mixture preferentially penetrate into the absorbent solids particles.

3. A device composed of a membrane filter structure of a waterproof polymer with solids particles incorporated, and containing one or more participants in a chemical reaction and, if appropriate, a detergent, which has been prepared by a process as claimed in either of claims 1 and 2.

4. The device as claimed in claim 3, wherein the incorporated solids particles are absorbent.

5. The device as claimed in claim 3, in a two-dimensional form.

6. The device as claimed in claim 3, wherein the proportion of the waterproof polymer is 3 to 50 g, preferably 6 to 35 g, per 100 g of device.

7. The device as claimed in claim 3, wherein the proportion of solids particles is 50 to 97 g, preferably 65 to 94 g, per 100 g of device.

8. The device as claimed in claim 3, containing 0 to 25 g of a detergent per 100 g of device.

## Revendications

1. Procédé de fabrication d'un dispositif, qui comprend une structure de filtre membranaire polymère hydrofuge comportant des particules solides incorporées et qui renferme un ou plusieurs participants à une réaction chimique et éventuellement un détergent, caractérisé en ce que les particules solides sont mélangées avec une solution de polymère formant filtre membranaire, les polymères étant dissous dans un système à solvants, qui contient des solvants de bas points d'ébullition pour le polymère, des agents gonflants à points d'ébullition plus élevés ainsi que des adjuvants de précipitation pour le polymère.

2. Procédé selon la revendication 1, caractérisé en ce que les parties précipitantes du mélange de solvants sont choisies de telle sorte que les parties précipitantes du mélange de solvants pénètrent de préférence dans les particules solides absorbantes.

3. Dispositif, qui comprend une structure de filtre membranaire en polymère hydrofuge comportant des particules solides incorporées et qui renferme un ou plusieurs participants à une réaction chimique et éventuellement un détergent, caractérisé en ce qu'il est fabriqué par un procédé selon l'une des revendications 1 et 2.

4. Dispositif selon la revendication 3, caractérisé en ce que les particules solides incorporées sont absorbantes.

5. Dispositif selon la revendication 3, caractérisé en ce qu'il est de forme plane .

6. Dispositif selon la revendication 3, caractérisé en ce que la proportion de polymère hydrofuge est de 3 à 50, de préférence de 6 à 35 g/100 g de dispositif.

7. Dispositif selon la revendication 3, caractérisé en ce que la proportion de particules solides est de 50 à 97, de préférence de 65 à 94 g/100 g de dispositif.

8. Dispositif selon la revendication 3, caractérisé en ce qu'il contient de 0 à 25 g d'un détergent pour 100 g de dispositif.